# EUROPÄISCHE PATENTSCHRIFT

(11) **EP 2 822 529 B1**
(45) Veröffentlichungstag und Bekanntmachung des Hinweises auf die Patenterteilung: **26.07.2017**
(21) Anmeldenummer: 13705823.6
(22) Anmeldetag: 26.02.2013
(51) Int. Cl.: A61K 8/02, A61K 8/37, A61K 8/92, A61K 8/25, A61Q 9/02

(54) **SILICIUMDIOXID IN RASIERPRODUKTEN**
SILICON DIOXIDE IN SHAVING PRODUCTS
DIOXYDE DE SILICIUM DANS DES PRODUITS DE RASAGE

(30) Priorität: 07.03.2012 DE 102012203565
(43) Veröffentlichungstag der Anmeldung: 14.01.2015
(73) Patentinhaber: Beiersdorf AG, 20253 Hamburg (DE)
(72) Erfinder: BELSER, Miriam, 22297 Hamburg (DE)
(74) Vertreter: Hartmann, Jost
(86) Internationale Anmeldenummer: PCT/EP2013/053810
(87) Internationale Veröffentlichungsnummer: WO 2013/131780

(56) Entgegenhaltungen:
- EP-A1- 0 829 259
- US-A- 5 587 156
- US-A1- 2003 114 572
- US-A1- 2005 106 196
- US-A1- 2010 247 914

## Beschreibung

Die Erfindung ist eine Rasurzubereitung umfassend 0,3 bis 5 Gew.% nichtkristallines, amorphes Siliciumdioxid SiO₂, welches nicht pyrogen hergestellt ist, mit einer Partikelgröße von 2 bis 30 µm.

Die Zubereitung führt aufgrund einer Rasurkraftreduktion zu einer hautfreundlicheren, gründlicheren Rasur.

Die Rasur des Barthaares zählt zur regelmäßigen Hygiene des Mannes. Benutzte man früher zur Rasur fast ausschließlich spezielle Rasiermesser, deren Handhabung einiges Geschick zur Vermeidung von Schnittverletzungen erforderte und deshalb überwiegend von Barbieren angewendet wurde, verwendet man heutzutage Klingenrasierer mit einer oder mehreren Klingen, deren Handhabung deutlich sicherer ist, oder Elektrorasierapparate.

Zur Erzielung einer angenehmen und gründlichen Rasur mit Hilfe eines Klingenrasierers benötigt man als Hilfsmittel Wasser und ein Rasierprodukt, eine Rasurzubereitung.

Diese Rasurzubereitungen liegen häufig als Schäume oder Gele vor und werden vor der Rasur auf die Haut aufgetragen.

Die Zubereitungen sollen das Haar auf die Rasur vorbereiten, es erweichen und einen Gleitfilm erzeugen, der das problemlose Gleiten der Klinge über die Haut ermöglicht.

Klassisches Rasierprodukt ist die Rasierseife, die mit einem Pinsel aufgeschäumt und in der Rasurzone verteilt wird. Neuere Produkte sind Rasierschäume und selbstschäumende Rasiergele.

Die Konstruktionen moderner Klingenrasierer schließen grobe Schnittverletzungen nahezu aus. Dennoch kann es zu Reizungen der Haut kommen, wenn die Klinge nicht optimal über die Haut gleitet. Ebenso kann es zu Mikroschnitten an Hautunebenheiten kommen, insbesondere im perifollikulären Bereich, unterstützt durch Zug- oder Biegevorgängen am zu rasierenden Haar. Der Wunsch nach einer möglichst gründlichen Rasur, also einem möglichst hautnahen Schnitt und einem Ausschluss von Irritationen, also einer Distanz von Klinge zur Haut, lässt sich dabei nur schwer vereinbaren.

Schlüssel für eine möglichst optimale Rasur ist das Rasierprodukt. Rasierprodukte erweichen das Haar, was den Schneidvorgang erleichtert und so zu weniger starkem Druck auf das Haar und somit zu weniger Ziepen führt. Bekannte Rasiermittel, Schäume und Gele, basieren auf basischen Zubereitungen, da bekanntermaßen die seifenbasierten und damit alkalischen Zubereitungen zu einem Quellen der Haaren führen, und damit eine gründlichere Rasur stattfinden kann.

Gleichzeitig bilden die Rasurzubereitungen einen Schmierfilm, der die Reibung herabsetzt und somit die Klinge sanft über die Haut gleiten lässt. Untersuchungen zeigen aber auch, dass ein zu weiches Haar dem Klingendruck nachgibt und durch Verbiegen ausweicht, was die Schnittebene von horizontal in Richtung vertikal verschiebt und somit zu ausgefransten Schnittkanten führt, was zulasten der Gründlichkeit geht. Ähnliches ist zu beobachten, wenn die Gleitwirkung auch auf dem Haar zu stark ist. Die Klinge gleitet am Haar ab, findet keinen Haltepunkt für einen glatten Schnitt, was häufig zu einem Abreißen des Haares, damit zu unangenehmen Empfinden, und durch diese ausgefransten Enden zu einer unsauberen, weniger gründlichen Rasur führt.

Den bekannten Rasurzubereitungen werden zuweilen Rasurhilfsmittel zugesetzt. Dabei handelt es sich um partikuläre Verbindungen oder wasserlösliche Polymere, wie PEG-14M, PEG-7M, PEG-90M (=PEG 90000), PEG 45M (Polymere des Ethylenoxids mit durchschnittlichen Polymerisationsgraden wie beziffert multipliziert mit Faktor 1000), Acrylate, PTFE (Polytetrafluorethylene) oder Cellulose-Derivate, die geeignet sind das Gleitvermögen zu verbessern.

US 6 342 211 B1 beschreibt den Vorgang des Abgleitens des Rasierers am Haar und das daraus folgende weniger gründliche Rasurergebnis. Zur Lösung dieses Problems wird 0,02 - 0,5% Polyacrylamid suspendiert in Wasser mit einem hohen Molekulargewicht von MW=14Mio. in einem Rasierprodukt eingesetzt, welches nach dem Prinzip eines Kugellagers eine optimale Gleitwirkung des Rasierers auf der Haut, aber nicht am Haar bewirkt.

US 5 756 081 und US 5 587 156 beschreiben Rasurzubereitungen, die feste, unlösliche, partikuläre Additive (z.B. PTFE, PE, Nylon, Silica u.a.) enthalten, welche der Klinge eine physikalische Unterstützung bieten, wodurch Schnitte verhindert werden und das Hautgefühl nach der Rasur verbessert wird.

EP 829 259 A1 beschreibt Rasierprodukte, die Mikropartikel (z.B. PA, Kaolin, PTFE, PV, SiO₂ etc.) enthalten. Diese Mikropartikel dienen als Peelingpartikel zur Entfernung von toten Hautzellen. Silikondioxidpartikel können dabei eine Partikelgröße von 7 bis 14 microns aufweisen.

EP 1 439 816 B1 beschreibt pyrogen hergestelltes Siliciumdioxid in kosmetischen Zubereitungen, wobei das SiO₂-Granulat als Träger für kosmetische Wirk- und/oder Hilfsstoffe wirkt.

US 2007 0031366 A2 beschreibt einen Prozess um eine gründlichere Rasur zu erhalten. Dafür wird die Haut u.a. mit einer Pre-Shave Zubereitung, die hautabschilfernde Partikel wie Silica, Metalloxide, PE etc. enthält, behandelt.

EP 1 704 897 B1 beschreibt nachschäumende Rasierprodukte bestehend aus Wasser, C10-C24-Alkancarbonsäure, Neutralisationsmittel, nicht-ionischem Tensid, hydrophobem Coemulgator, C3-C9 Polyol, verdickendes Polymer, das kein PEG darstellt, PEG mit mittlerem Molekulargewicht von mind. 300.000 Dalton und Kieselsäure. Durch die Verringerung des Gehaltes an hochmolekularen PEGs sowie den Zusatz an Kieselsäure zusammen mit einer spezifischen Kombination aus Seifen, nichtionischen Tensiden, hydrophoben Coemulgatoren, Polyolen und Polymerverdickern sollen die Anwendungseigenschaften, das Hautgefühl, die Schaumeigenschaften sowie die Produktions-und Abfülleigenschaften verbessert werden.

Wünschenswert ist es eine gründliche Rasur bei möglichst geringen oder besser gar keinen Irritationen der Haut zu erreichen.

Dabei sollte möglichst ein hautnaher Schnitt des Haares erreicht werden, wobei das Abgleiten der Rasierklinge am Haar zu vermeiden ist und damit weniger vertikale Schnitte erfolgen, so dass die Haare weniger ausgefranste Enden aufweisen.

Die Erfindung ist eine Rasurzubereitung mit mindestens einer Ölphase, die nichtkristallines, amorphes Siliciumdioxid SiO₂ umfasst, welches nicht pyrogen hergestellt ist, mit einer Partikelgröße von 2 bis 30 µm.

Der Gewichtsanteil des nicht pyrogen hergestellten Siliciumdioxids beträgt 0,3 bis 5 Gew.%, bezogen auf die Gesamtmasse der Zubereitung, bevorzugt 0,4 bis 1,2 Gew.%.

Der Anteil an Siliciumdioxid bezogen auf die Gesamtmasse der Zubereitung mit Teibmittel ist bevorzugt 0,4 bis 1 Gew.%. Der Anteil an Siliciumdioxid bezogen auf die Gesamtmasse der Zubereitung ohne Treibmittel ist bevorzugt 0,5 bis 1,2 Gew.%.

Es wurde überraschend festgestellt, dass Rasierprodukte, die erfindungsgemäßes Siliciumdioxid mit dieser Partikelgröße enthalten, den Nachteil des Standes der Technik nicht zeigen.

Die erfindungsgemäße Rasurzubereitung bildet aufgrund des in der Ölphase enthaltenen SiO₂ eine partikuläre Gleitschicht aus und ermöglicht eine optimal gründliche Rasur ohne Hautirritationen. Die erfindungsgemäßen SiO₂-Partikel ermöglichen, ähnlich einem Kugellagerprinzip, ein Gleiten der Klinge auf der Haut. Entgegen den aus dem Stand der Technik bekannten Füllstoffen können diese Partikel aus geometrischen Gründen keine Gleitschicht auf dem Haar ausbilden und die Klinge findet sofortigen Halt für einen gründlichen Schnitt.

Rasiermittel sind Trocken- oder Nassrasurapparate, metallische Klingen oder Messer.

Um eine Aussage über die Rasurgründlichkeit als auch Eignung eines Rasierproduktes treffen zu können lässt sich die Rasurkraft ermitteln.

Bei der Rasurkraft handelt es sich um eine Kombination von Schneid- und Gleitvorgängen. Das Rasierprodukt wird auf eine präparierte Schweinehaut aufgetragen und anschließend wird der Rasierer (das Rasiermittel), der auf einem Adapter aufgebracht ist und mit einer Standard-Prüfmaschine verbunden ist, über die Schweinehaut gezogen. Dabei werden die (Bart)Haare geschnitten und die dazu benötigte Kraft aufgezeichnet (1. Rasurkraft). Dieser Vorgang wird danach einmal wiederholt (2. Rasurkraft). Anschließend wird die Schweinehaut mit einem separaten Rasierer gründlich von den eventuell verbliebenen Stoppeln befreit und erneut Produkt aufgetragen. Der Rasierer gleitet nun über die glatte Haut, woraus sich Rückschlüsse auf die Gleiteigenschaften des Produktes ziehen lassen (Gleitkraft).

Eine kosmetische Zubereitung, eine Rasurzubereitung, vermag die Gleit- und Schneidkräfte dabei zu beeinflussen.

In einer in-vitro Rasurkraft-Messung auf Schweinehaut wurden 3 Rasierprodukte miteinander verglichen. Alle drei Zubereitungen umfassen 0,5 Gew.% eines Polymers bzw. eines Partikels (Nylon-12, Siliciumdioxid (Silica Pearl P-4), Polyethylen (Micropoly 1160S)).

Die Testprodukte sind in der nachfolgenden Tabelle 1 aufgeführt.

**Tabelle 1:**

| INCI | Testprodukt 1 (10) m[%] | Testprodukt 2 (20) m[%] | Testprodukt 3 (30) m[%] |
|---|---|---|---|
| Stearic Acid | 5,50 | 5,50 | 5,50 |
| Propylene Glycol | 1,00 | 1,00 | 1,00 |
| Aqua | ad 100 | ad 100 | ad 100 |
| Persea Gratissima Oil | 0,10 | 0,10 | 0,10 |
| Laureth-23 | 2,75 | 2,75 | 2,75 |
| Nylon-12 | 0,50 | | |
| SILICA PEARL P-4 | | | 0,50 |
| MICROPOLY 1160S | | 0,50 | |
| Glycerin | 2,48 | 2,48 | 2,48 |
| Kaliumhydroxid | 0,17 | 0,17 | 0,17 |
| Hydroxypropyl Methylcellulose | 0,15 | 0,15 | 0,15 |
| Gossypium Herbaceum Seed Oil | 0,40 | 0,40 | 0,40 |
| Coco-Caprylate/Caprate | 0,50 | 0,50 | 0,50 |
| Piroctone Olamine | 0,10 | 0,10 | 0,10 |
| Triethanolamine | 2,79 | 2,79 | 2,79 |
| PEG-7M | 0,05 | 0,05 | 0,05 |
| Bisabolol | 0,20 | 0,20 | 0,20 |
| Parfum | 0,30 | 0,30 | 0,30 |

Die Zubereitung wird anschließend mit Treibgas versetzt:

| | | | |
|---|---|---|---|
| Isobutane | 2,88 | 2,88 | 2,88 |
| Propane | 0,92 | 0,92 | 0,92 |
| Butane | 0,20 | 0,20 | 0,20 |

Testprodukt 05 ist reines Wasser.

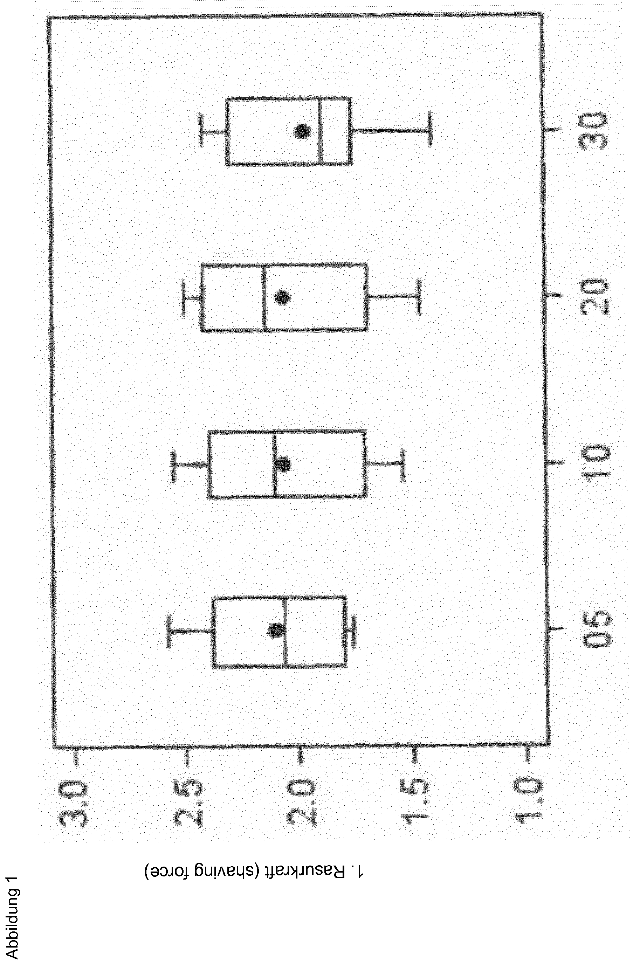

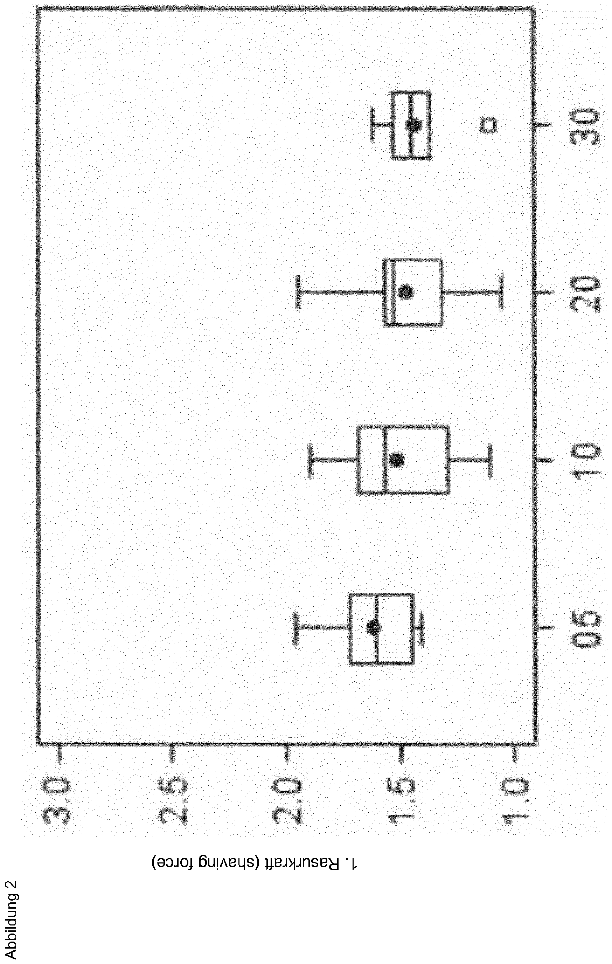

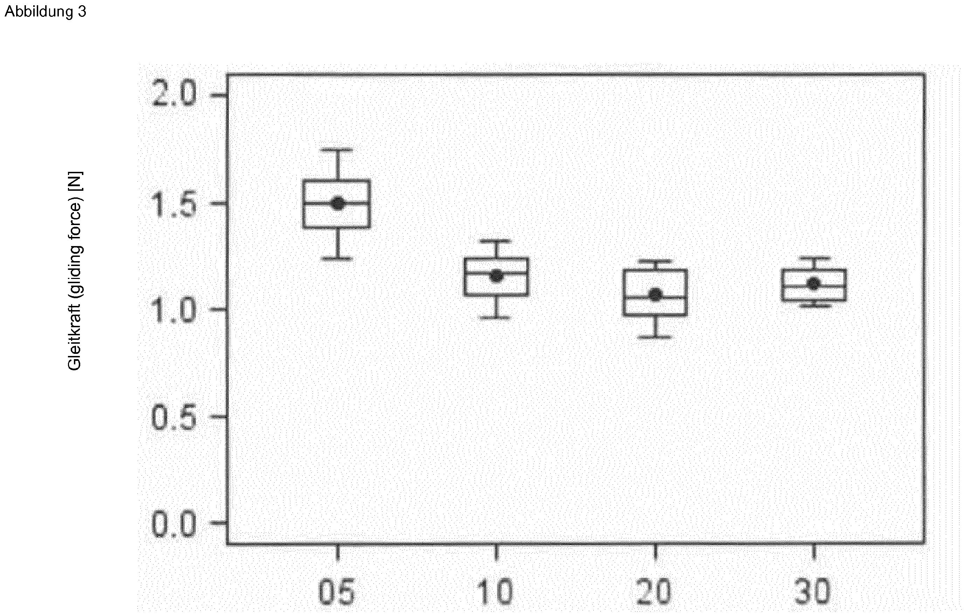

In Abbildungen 1 und 2 sind die 1. bzw. 2. Rasurkräfte und in Abbildung 3 die Gleitkräfte der Testprodukte dargestellt. Nachfolgende Tabelle erläutert die Messergebnisse.

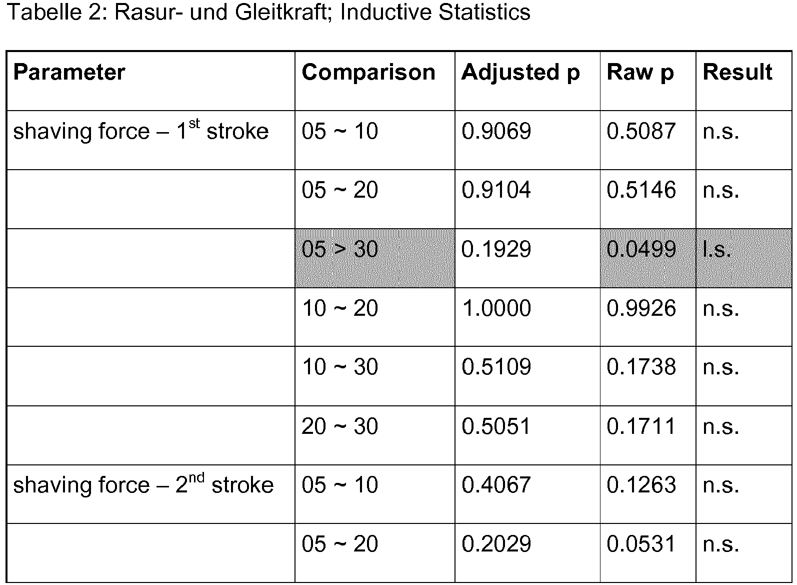

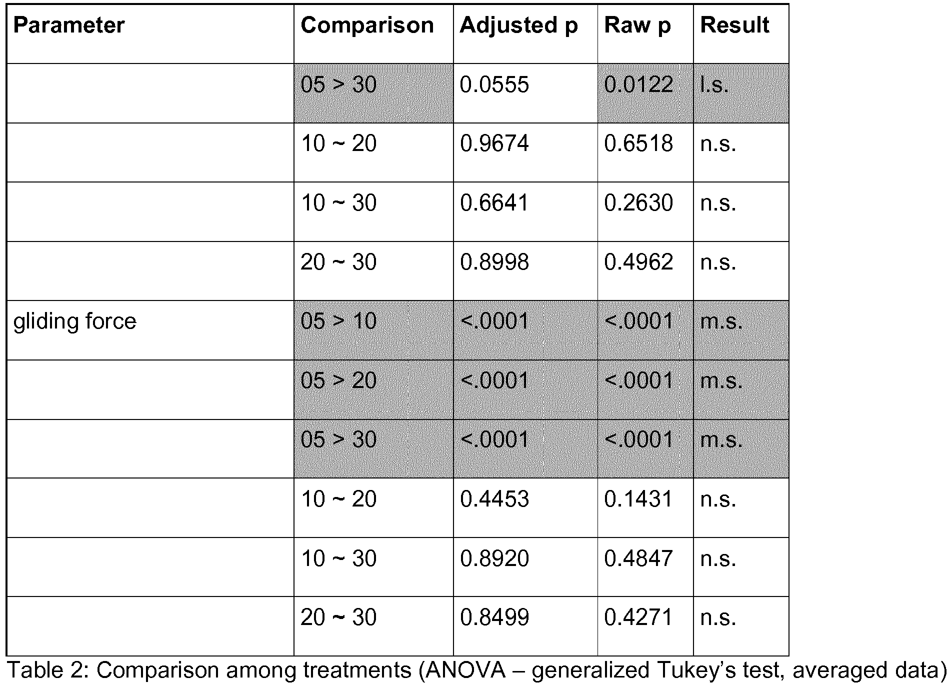

Überraschenderweise zeigt sich, dass erfindungsgemäßes Siliciumdioxid (Silica Pearl P-4) die Rasurkraft im Vergleich zu Wasser signifikant und im Vergleich zu Polyethylen und Nylon-12 deskriptiv erniedrigt. In den Gleiteigenschaften schneiden alle 3 Produkte signifikant besser ab als Wasser, wobei es zwischen den Produkten keine signifikanten Unterschiede gibt.

Das erfindungsgemäße Siliciumdioxid bildet in der Rasierzubereitung auf der Haut eine partikuläre Gleitschicht aus, die ähnlich dem Kugellagerprinzip ein Gleiten der Klinge auf der Haut ermöglicht. Da die Siliciumdioxid Partikel aufgrund ihrer geometrisch sehr runden Form jedoch keine Gleitschicht auf dem Haar ausbilden können, findet die Klinge einen sofortigen Halt für einen optimalen Schnitt. Dadurch sind die notwendigen Schneidkräfte geringer und die Rasur somit angenehmer. Erfindungsgemäßes Siliciumdioxid hat somit einen positiven Einfluss auf die Rasurkräfte im Rasurprozess.

Die erfindungsgemäße Zubereitung lässt sich somit zur Erniedrigung der Rasurkraft nach topischer Applikation der Zubereitung verwenden.

In einem in-vivo Paneltest, bei dem sich geschulte Probanden nach einem standardisierten Verfahren rasieren, wurde ein Rasierprodukt mit erfindungsgemäßen Siliciumdioxid gegen ein Rasierprodukt mit Nylon-12 verglichen. Die Zubereitung mit erfindungsgemäßem Siliciumdioxid zeigt eine signifikant höhere Dämpfung.

Der Parameter Dämpfung ist definiert als die Produktmenge, die zwischen Klinge und Haut spürbar ist, also den Rasierer abdämpft vor dem Hautkontakt. Je höher die Dämpfung, desto mehr Produkt befindet sich zwischen Klinge und Haut, desto höher ist der Rasierkomfort.

Weiterhin lässt sich beobachten, dass die erfindungsgemäße Zubereitung vorteilhaft einen signifikant höheren Film auf der Haut hinterlässt.

Nummerisch ist nach der Rasur bei nasser Haut das Gleiten der Finger über die Haut höher. Das Schaben der Klinge (Geräusch) ist nummerisch geringer.

Überraschender Weise ist die Dämpfung mit erfindungsgemäßer Zubereitung höher, obwohl die SiO₂-Partikel mit durchschnittlich 10 µm Partikelgröße nur halb so groß sind wie die Nylon-12 Partikel mit durchschnittlich 20 µm. Man würde also bei den kleineren Partikeln eine geringere Dämpfung erwarten, da mit kleineren Partikeln der Abstand der Klinge zur Haut geringer ist.

Folgende Zubereitungen der Tabelle 3 wurden im in-vivo Test untersucht:

**Tabelle 3: In-vivo Paneltest - Dämpfung**

| | Testprodukt 1 | Testprodukt 2 |
|---|---|---|
| INCI | m [%] | m [%] |
| Stearic Acid | 5,50 | 5,50 |
| Propylene Glycol | 1,00 | 1,00 |
| Aqua | ad 100 | ad 100 |
| Persea Gratissima Oil | 0,10 | 0,10 |
| Laureth-23 | 2,75 | 2,75 |
| Nylon-12 | 0,50 | |
| Siliciumdioxid | | 0,50 |
| Glycerin | 2,48 | 2,48 |
| Kaliumhydroxid | 0,17 | 0,17 |
| Hydroxypropyl Methylcellulose | 0,15 | 0,15 |
| Gossypium Herbaceum Seed Oil | 0,40 | 0,40 |
| Coco-Caprylate/Caprate | 0,50 | 0,50 |
| Piroctone Olamine | 0,10 | 0,10 |
| Triethanolamine | 2,79 | 2,79 |
| PEG-7M | 0,05 | 0,05 |
| Bisabolol | 0,20 | 0,20 |
| Parfum | 0,30 | 0,30 |
| Die Zubereitung wird anschließend mit Treibmittel versetzt: | | |
| Propane | 0,92 | 0,92 |
| Butane | 0,20 | 0,20 |
| Isobutane | 2,88 | 2,88 |

Die Bewertung erfolgte durch trainierte Probanden. Die Bewertung erfolgt anhand einer Skala von 0 bis 10 in 0,5er-Schritten. Für jedes Attribut ist ein Standardwert definiert. Je höher der Wert, desto stärker ist das Attribut ausgeprägt.

Nachfolgende Tabelle 4 zeigt die Testergebnisse der Dämpfungsuntersuchung im Vergleich auch zu einem Marktprodukt.

**Tabelle 4: in-vivo Paneltestergebnisse**

| | Mittelwert | Mittelwert | | Signifikanz (> 95%) | Signifikanz (> 90%) |
|---|---|---|---|---|---|
| | Testprodukt 1 0,5% Nylon-12 | Testprodukt 2 0,5% Silicapearl P4 (SiO2) | Standardwerte Nivea Sensitiv Rasierschaum | | |
| **Im Uhrglas** | | | | | |
| Formstabilität | 7,8 | 7,7 | 8 | | |
| Festigkeit | 5,0 | 5,1 | 5 | | |
| **Im Gesicht** | | | | | |
| Verteilbarkeit | 7,0 | 6,9 | 7 | | |
| Gleichmässigkeit | 7,8 | 7,9 | 8 | | |
| Kühleffekt | 1,0 | 1,1 | 1 | | |
| Fädenziehen | 1,1 | 1,0 | 1 | | |
| Klebrigkeit | 5,0 | 4,7 | 5 | | |
| Spitzenbildung | 4,8 | 4,9 | 5 | | |
| **Während der Rasur** | | | | | |
| Gleiten | 6,0 | 6,0 | 6 | | |
| Dämpfung | 6,0 | 6,3 | 6 | ++ | |
| Klinge Schaben | 3,5 | 3,4 | 3,5 | | |
| Abspülbarkeit | 7,9 | 7,6 | 8 | | |
| **Nach der Rasur-nass** | | | | | |
| Haut Gleiten | 5,9 | 6,2 | 6 | | |
| Film auf der Haut | 1,7 | 2,1 | 2 | | + |
| **Nach der Rasur trocken** | | | | | |
| Stoppeln | 1,9 | 1,9 | 2 | | |
| Hautspannung | 1,5 | 1,4 | 1,5 | | |
| Kühleffekt | 1,2 | 1,2 | 1 | | |
| Geschmeidigkeit | 5,9 | 5,9 | 6 | | |

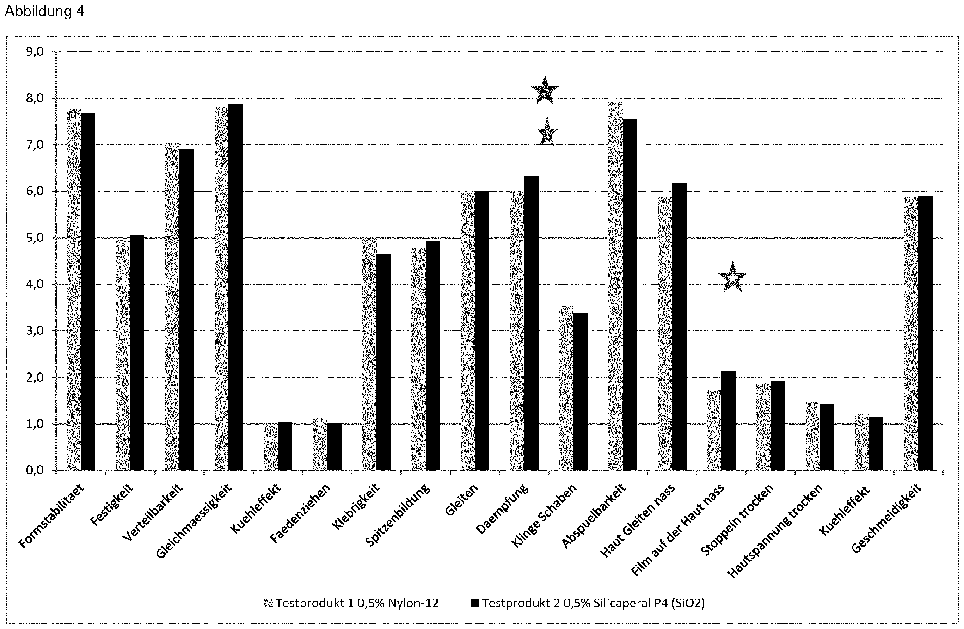

Die Ergebnisse des Paneltests sind in Abbildung 4 dargestellt.

In einem home-in-use Test mit 100 männlichen Verbrauchern wurden 2 Rasierprodukte mit 0,5 Gew.% erfindungsgemäßen Siliciumdioxid und 0,5 Gew.% Nylon-12 gegen ein handelsübliches Rasierprodukt ohne partikuläre Additive getestet. Die erfindungsgemäße Zubereitung schnitt in den Parametern: "Haut fühlt sich weich und geschmeidig an" und "Rasur war gründlich" sowie in der Gesamtbeurteilung signifikant besser ab. Nummerisch war die erfindungsgemäße Zubereitung mit Siliciumdioxid außerdem besser in "Schaum lässt sich gut auf der Haut verteilen", "Schaum haftet gut im Gesicht", "Haut spannt nicht", "Barthaar wird optimal auf die Rasur vorbereitet".

Die Tatsache, dass die erfindungsgemäßen Zubereitungen mit Siliciumdioxid in der Gründlichkeit deutlich besser sind, kann auf das sog. "Kugellagerprinzip" zurückgeführt werden. Dies wiederum basiert auf der erfindungsgemäßen Partikelgröße der gewählten Siliciumdioxidpartikel. Da sich aufgrund der besonderen geometrischen Form der erfindungsgemäßen SiO2 Partikel keine partikuläre Gleitschicht am Haar aufbaut findet die Klinge so einen sofortigen Halt am Haar. Dadurch wird das Haar sauber geschnitten ohne ausgefranste Enden, die zulasten der Gründlichkeit gehen würden.

Die im Stand der Technik bekannten Zubereitungen (z.B. US 6342211 B1) umfassen sehr große, in Wasser suspendiert vorliegende, Polyacrylamid-Moleküle, die nur eine Schmierung der Haut, aber nicht des Haares erreichen.

Erstaunlicherweise zeigen die erfindungsgemäßen Zubereitungen denselben Effekt, wobei die SiO₂ Partikel aber im Gegensatz sehr kleine feste Partikel mit einer durchschnittlichen Größe von insbesondere vorteilhaft 5 - 10 µm handelt. Dies ist mit den bisherigen Erkenntnissen zur Rasur- und Gleiteigenschaft von Rasiermitteln und -produkten überraschend.

Weiterer entscheidender Unterschied ist, dass erfindungsgemäß die SiO₂ Partikel in der Ölphase vorliegen. Wenn die Partikel in der Ölphase vorliegen, was bei einer O/W - Emulsionszubereitung der inneren, diskontinuierlichen Phase entspricht, ist ein Einfluss auf das Haar, beispielsweise die "Schmierung" des Haares, unwahrscheinlicher als wenn die Partikel in der Wasserphase vorliegen würden. Die unterstützende Gleitwirkung für die Klinge der Rasiermittel ist aber dennoch vorhanden und führt zu den vorteilhaften Eigenschaften.

Ein Vorteil dabei ist, dass sich die SiO₂ Partikel in die Ölphase leichter einarbeiten lassen.

Weiterhin konnte In-vitro und in-vivo gezeigt werden, dass beispielsweise übliche Partikel aus PE oder Nylon-12 nicht den gleichen positiven Effekt haben wie erfindungsgemäßes Siliciumdioxid. Ebenso sind die im Stand der Technik aufgeführten Partikel mit einer Partikelgröße von 50 - 200 µm deutlich größer als erfindungsgemäßes Siliciumdioxid.

Dabei ist bekannt, dass Partikel ab einer Größe von mehr als 40 µm als Peelingpartikel auf der Haut spürbar sind. Demnach dürften solch große Partikel in einem Rasierprodukt eher unangenehm während der Anwendung sein. Auch wird die Gründlichkeit deutlich verringert, da der Abstand der Klinge zur Haut durch die Partikelgröße zu sehr vergrößert wird.

Siliciumdioxid ist eine Sammelbezeichnung für die Modifikationen der Oxide des Siliciums mit der Summenformel SiO₂. Im deutschen Sprachraum wird mitunter fälschlich für Siliciumdioxid die Bezeichnung Kieselsäure benutzt. Erfindungsgemäß wird unter Siliciumdioxid keine Kieselsäure oder Fällungskieselsäure verstanden, wie sie im Stand er Technik erwähnt wird.

Synthetisches SiO₂, das amorph vorliegt, wird großtechnisch in unterschiedlichen Prozessen in großen Mengen erzeugt.

Eine Herstellungsvariante ist die Erzeugung von so genanntem pyrogenen SiO₂ in einer Knallgasflamme, ausgehend von flüssigen Chlorsilanen wie Siliciumtetrachlorid (SiCl₄).

Bei der Herstellung von pyrogenem SiO₂ können sich beim Abkühlen größere Agglomerate bilden, die eine eher kettenförmige Struktur aufweisen. D.h. bei der pyrogenen Herstellung von Siliciumdioxid ist die erfindungsgemäß bevorzugte Kugelform des Siliciumdioxids nicht gewährleistet. Erfindungsgemäß werden daher diese Formen des Siliciumdioxids, pyrogenes SiO₂, ausgeschlossen.

Bei dem erfindungsgemäßen Siliciumdioxid handelt es sich vielmehr um nichtkristallines, amorphes Siliciumdioxid SiO₂, welches nicht pyrogen hergestellt ist.

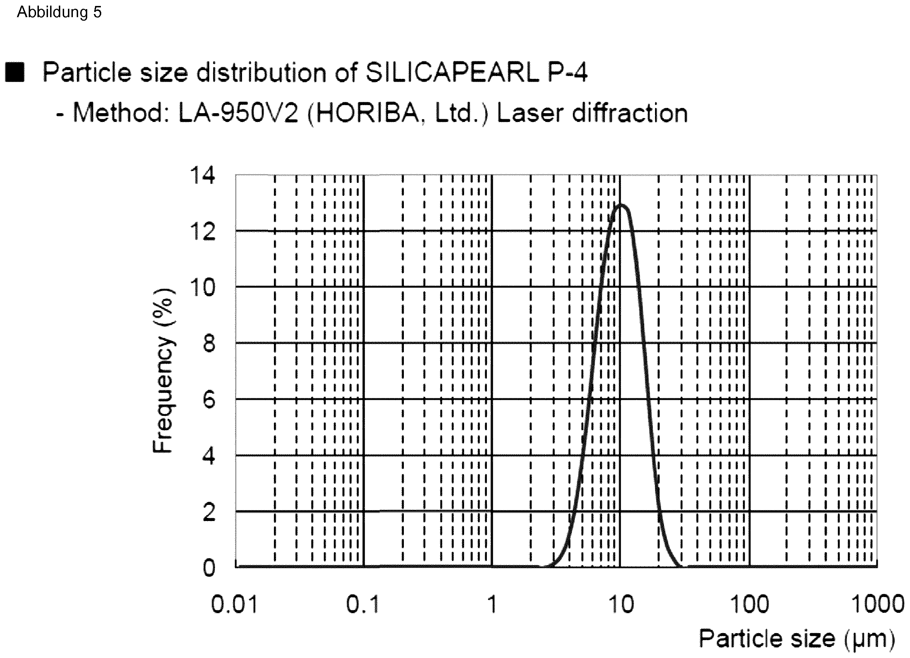

Eine weitere besondere Eigenschaft der erfindungsgemäßen SiO₂ Partikel liegen in der engen Partikelgrößenverteilung, wie sie in Abbildung 5 dargestellt ist.

Das erfindungsgemäße SiO₂ weist eine Partikelgröße von 2 bis 30 µm, insbesondere 5 bis 15 µm, insbesondere im Bereich um 10 µm auf. Der Bereich um 10 µm ist als Bereich von 9,0 bis 12,0 µm, vorteilhaft als Bereich von 9,5 bis 11,0 µm zu verstehen.

Als erfindungsgemäßes SiO₂ kann beispielsweise der Rohstoff Silica Pearl P-4 von JGC verwendet werden.

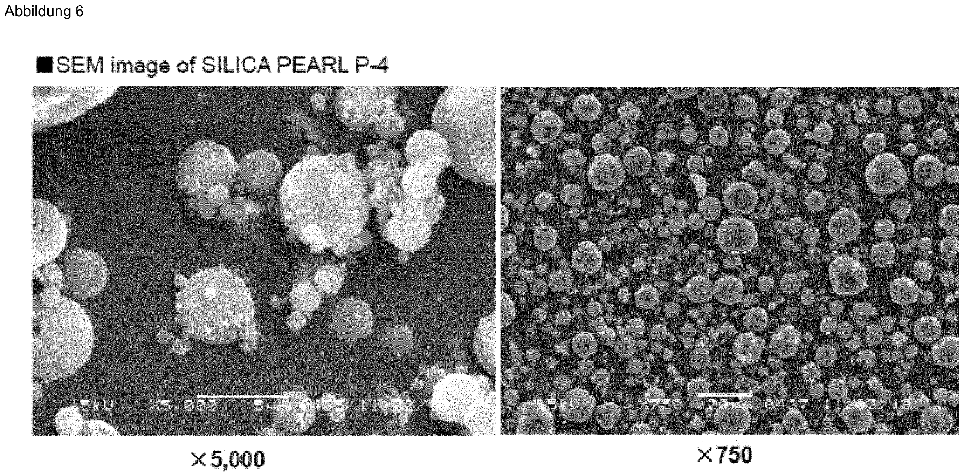

Weitere Charakteristik der erfindungsgemäßen SiO₂-Partikel liegen in ihrer runden geometrischen Form, wie es in Abbildung 6 dargestellt ist.

Es wurden viele verschiedene Techniken für die Bestimmung der Partikelgrößenverteilung entwickelt, in vielen verschiedenen Industriebereichen jedoch ist die Laserbeugung die bevorzugte Methode.

Die Laserbeugung oder LALLS (Low Angle Laser Light Scattering)-Methode kann zur zerstörungsfreien Analyse von nassen und trockenen Proben von Partikeln mit einer Größe von 0,02 bis 2000 Mikrometer verwendet werden und besitzt besondere Vorteile, die sie bei vielen Materialien von anderen Methoden positiv unterscheidet.

Die Definition des Begriffs "Partikelgrößenverteilung" ist wie folgt zu verstehen. Partikel sind dreidimensionale Gebilde. Drei Parameter (Länge, Breite, Höhe) sind erforderlich, um eine vollständige Beschreibung von Partikeln geben zu können. Es ist also schwer möglich, ein Partikel durch Angabe einer einzigen Zahl zu beschreiben, die der Partikelgröße entspricht. Bei der Mehrzahl der Methoden zur Größenbestimmung wird deshalb davon ausgegangen, dass das zu messende Material kugelförmig ist, da eine Kugel die einzige Form ist, die mittels einer einzigen Zahl (dem Durchmesser) beschrieben werden kann.

Besteht das Produkt aus kugelförmigen Partikeln, so ist die Partikelgröße durch die Angabe des Kugeldurchmessers eindeutig definiert, wie erfindungsgemäß bevorzugt im Bereich von 5 bis 15 µm. Die Mehrzahl der feinkörnigen Materialien besteht aber nicht aus Kugeln, sondern aus mehr oder weniger unregelmäßig geformten Partikeln, die in einem Extremfall nadelförmig, im anderen plättchenförmig sein können. Die Dispersitätseigenschaft der Einzelpartikeln lässt sich hier durch das Partikelvolumen und zusätzliche Parameter wie Sphärizität (Kugelähnlichkeit) beschreiben. Anstelle der für Kugeln einparametrigen Funktion erhält man eine vielparametrige Dispersitätsfunktion, deren Bestimmung mit großem messtechnischem Aufwand verbunden ist. Ein derartiger Aufwand ist nur dann gerechtfertigt, wenn er dazu dient, wesentliche Informationen über Produkteigenschaften zu gewinnen.

Daher beschränkt man sich in der Praxis normalerweise und auch erfindungsgemäß auf die Angabe einer einparametrigen Verteilungsfunktion für die Partikelgröße.

Die erfindungsgemäßen Zubereitungen sind bevorzugt emulsionsbasierende Zubereitungen, Gele, Schäume oder Creme. Auch als Lotion, Spray oder Aerosol lassen sich die erfindungsgemäßen Zubereitungen bereit stellen.

Die dazu jeweils notwendigen Begleitsubstanzen, wie Treibgase bei Aerosolen, kann der Fachmann aus den bekannten Mitteln wählen.

Als Emulsionen sind vorteilhaft und enthalten z.B. Fette, Öle, Wachse und anderen Fettkörper, sowie Wasser und einen Emulgator, wie er üblicherweise für einen solchen Typ der Formulierung verwendet wird.

Vorteilhaft sind die erfindungsgemäßen Zubereitungen Rasierschäume, Rasiergele oder Rasiercreme.

Erfindungsgemäße Zubereitung umfasst eine Öl- oder Lipidphase.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Zubereitung, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft umfasst die Ölphase ein oder mehrere Lipide gewählt aus Persea Gratissima Oil, Gossypium Herbaceum Seed Oil, Coco-Caprylate/Caprat und/oder Isopropyl Palmitat.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse, sofern nicht ausgeschlossen, oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Moisturizer können vorteilhaft auch als Antifaltenwirkstoffe zum Schutz vor Hautveränderungen, wie sie z. B. bei der Hautalterung auftreten, verwendet werden.

Die Herstellung erfindungsgemäßer Zubereitungen erfolgt gemäß nach üblichen Herstellanweisungen. Die Einarbeitung der SiO₂-Partikel erfolgt beispielsweise unter Energieeintrag mit einem Ultraturrax (Labormaßstab) oder über ein starkes Rührwerk im Kessel (Produktion) in die Fettphase der Zubereitung. SiO₂ Partikel sind nicht wasserlöslich, daher ist eine Dispergierung in der Ölphase vorteilhaft.

Die nachfolgenden Beispiele illustrieren die erfindungsgemäßen Zubereitungen. Die Angaben beziehen sich auf Gewichtsanteile bezogen auf die Gesamtmasse der jeweiligen Zubereitung ohne bzw. mit Treibmittel.

**Beispiel Rasierschäume**

| INCI | Bsp 1 | Bsp 2 |
|---|---|---|
| | m [%] | m [%] |
| Stearic Acid | 5,50 | 5,50 |
| Propylene Glycol | 1,00 | |
| Aqua | ad 100 | ad 100 |
| Persea Gratissima Oil | 0,10 | |
| Laureth-23 | 2,75 | 2,75 |
| Siliciumdioxid* | 0,50 | 0,50 |
| Glycerin | 2,48 | 2,60 |
| Kaliumhydroxid | 0,17 | 0,17 |
| Hydroxypropyl Methylcellulose | 0,15 | 0,15 |
| Gossypium Herbaceum Seed Oil | 0,40 | |
| Coco-Caprylate/Caprate | 0,50 | 0,50 |
| Piroctone Olamine | 0,10 | 0,10 |
| Triethanolamine | 2,79 | 2,79 |
| PEG-7M | 0,05 | 0,05 |
| Bisabolol | 0,20 | |
| Maltodextrin + Chamomilla Recutita Flower Extract | | 0,01 |
| Parfum | 0,30 | 0,30 |

96 Gew.% der obigen Zubereitung, bezogen auf die Gesamtmasse mit Treibmittel, wird mit Treibmittel wie folgt versetzt:

| | | |
|---|---|---|
| Isobutane | 2,88 | 2,88 |
| Propane | 0,92 | 0,92 |
| Butane | 0,20 | 0,20 |

**Beispiele Rasiergele**

| | Bsp 3 | Bsp 4 |
|---|---|---|
| INCI | m [%] | m [%] |
| Isopropyl Palmitate | | 1,50 |
| Propylene Glycol | 0,50 | |
| Aqua | ad 100 | ad 100 |
| Tocopheryl Acetate | | 0,01 |
| Paraffinum Liquidum | 1,50 | |
| BHT | 0,03 | 0,03 |
| Polyisobutene | 0,50 | 0,50 |
| Siliciumdioxid* | 0,50 | 0,50 |
| Glycerin | 2,48 | 2,48 |
| Hydroxyethylcellulose | 0,40 | 0,40 |
| Hydroxypropyl Methylcellulose | 0,10 | 0,10 |
| Palmitic Acid | 8,40 | 8,40 |
| Oleth-20 | 6,00 | 6,00 |
| PEG-14 M | 0,10 | 0,10 |
| Gossypium Herbaceum Seed Oil | 0,50 | |
| Piroctone Olamine | 0,10 | 0,10 |
| PEG-90 Glyceryl Isostearate | 0,35 | 0,35 |
| Laureth-2 | 0,04 | 0,04 |
| Triethanolamine | 6,00 | 6,00 |
| Bisabolol | 0,20 | |
| Parfum | 0,40 | 0,20 |
| Maltodextrin + Chamomilla Recutita Flower Extract | | 0,01 |

95 Gew.% der obigen Zubereitung, bezogen auf die Gesamtmasse mit Treibmittel, wird mit Treibmittel wie folgt versetzt:

| | | |
|---|---|---|
| Isopentane | 3,75 | 3,75 |
| Isobutane | 1,25 | 1,25 |

| | | |
|---|---|---|
| * nichtkristallines, amorphes Siliciumdioxid SiO₂, nicht pyrogen hergestellt. | | |

## Patentansprüche

1. Rasurzubereitung mit mindestens einer Ölphase **dadurch gekennzeichnet, dass** die Ölphase nichtkristallines, amorphes Siliciumdioxid SiO₂ umfasst, welches nicht pyrogen hergestellt ist, mit einer Partikelgröße 2 bis 30 µm, wobei der Gewichtsanteil des Siliciumdioxids 0,3 bis 5 Gew.% beträgt, bezogen auf die Gesamtmasse der Zubereitung.

2. Zubereitung nach Anspruch 1 **dadurch gekennzeichnet, dass** das Siliciumdioxid eine Partikelgröße von 5 bis 15 µm, insbesondere um 10 µm, aufweist.

3. Zubereitung nach Anspruch 1 **dadurch gekennzeichnet, dass** der Anteil an Siliciumdioxid 0,4 bis 1,2 Gew.% beträgt, bezogen auf die Gesamtmasse der Zubereitung.

4. Zubereitung nach einem der vorstehenden Ansprüche **dadurch gekennzeichnet, dass** die Siliciumdioxidpartikel eine runde Form aufweisen.

5. Zubereitung nach einem der vorstehenden Ansprüche **dadurch gekennzeichnet, dass** die Ölphase ein oder mehrere Lipide gewählt aus der Gruppe Persea Gratissima Oil, Gossypium Herbaceum Seed Oil, Coco-Caprylate/Caprat und/oder Isopropyl Palmitat umfasst.

6. Verwendung einer Zubereitung nach einem der vorstehenden Ansprüche zur Erniedrigung der Rasurkraft nach topischer Applikation der Zubereitung.

## Claims

1. Shaving preparation with at least one oil phase, **characterized in that** the oil phase comprises noncrystalline, amorphous silicon dioxide SiO₂, which has not been produced by pyrogenic means, having a particle size of 2 to 30 µm, where the weight fraction of the silicon dioxide is 0.3 to 5% by weight, based on the total mass of the preparation.

2. Preparation according to Claim 1, **characterized in that** the silicon dioxide has a particle size of from 5 to 15 µm, in particular around 10 µm.

3. Preparation according to Claim 1, **characterized in that** the fraction of silicon dioxide is 0.4 to 1.2% by weight, based on the total mass of the preparation.

4. Preparation according to any one of the preceding claims, **characterized in that** the silicon dioxide particles have a round shape.

5. Preparation according to any one of the preceding claims, **characterized in that** the oil phase comprises one or more lipids selected from the group Persea Gratissima Oil, Gossypium Herbaceum Seed Oil, Coco-Caprylate/Caprate and/or Isopropyl Palmitate.

6. Use of a preparation according to any one of the preceding claims for reducing the shaving force following topical application of the preparation.

## Revendications

1. Préparation de rasage contenant au moins une phase huileuse, **caractérisée en ce que** la phase huileuse comprend du dioxyde de silicium SiO₂ amorphe non cristallin, qui n'est pas fabriqué par pyrogénation, ayant une taille de particule de 2 à 30 µm, la proportion en poids du dioxyde de silicium étant de 0,3 à 5 % en poids, par rapport à la masse totale de la préparation.

2. Préparation selon la revendication 1, **caractérisée en ce que** le dioxyde de silicium présente une taille de particule de 5 à 15 µm, notamment d'environ 10 µm.

3. Préparation selon la revendication 1, **caractérisée en ce que** la proportion de dioxyde de silicium est de 0,4 à 1,2 % en poids, par rapport à la masse totale de la préparation.

4. Préparation selon l'une quelconque des revendications précédentes, **caractérisée en ce que** les particules de dioxyde de silicium présentent une forme ronde.

5. Préparation selon l'une quelconque des revendications précédentes, **caractérisée en ce que** la phase huileuse comprend un ou plusieurs lipides choisis dans le groupe constitué par Persea Gratissima Oil, Gossypium Herbaceum Seed Oil, le coco-caprylate/caprate et/ou le palmitate d'isopropyle.

6. Utilisation d'une préparation selon l'une quelconque des revendications précédentes pour la réduction du pouvoir rasant après application topique de la préparation.
